# EUROPEAN PATENT SPECIFICATION

(11) **EP 1 722 229 B1**
(45) Date of publication and mention of the grant of the patent: **07.10.2009**
(21) Application number: 05701698.2
(22) Date of filing: 03.02.2005
(51) Int. Cl.: G01N 33/564, G01N 33/566, C07K 14/705

(54) **METHOD OF ASSESSING RISK OF AND PREDISPOSITION TO THE DEVELOPMENT OF A PATHOLOGY RELATED TO THE PRESENCE OF ANTI-EPCR ANTIBODIES**
VERFAHREN ZUR BEURTEILUNG DER GEFAHR UND VERANLAGUNG, EINE MIT DEM VORLIEGEN VON ANTI-EPCR-ANTIKÖRPERN VERBUNDENE SYMPTOMATIK ZU ENTWICKELN
METHODE D'EVALUATION DU RISQUE ET DE LA PREDISPOSITION A DEVELOPPER UNE PATHOLOGIE ASSOCIEE A LA PRESENCE D'AUTOANTICORPS DU EPCR

(30) Priority: 06.02.2004 ES 200400269
(43) Date of publication of application: 15.11.2006
(73) Proprietor: Proyecto de Biomedicina Cima, S.L., 31003 Pamplona (Navarra) (ES)
(72) Inventor: HERMIDA SANTOS, José, 31008 Pamplona (Navarra) (ES); MONTES DIAZ, Ramon, 31008 Pamplona (Navarra) (ES); HURTADO LINARES, Verónica, 31008 Pamplona (Navarra) (ES)
(74) Representative: ABG Patentes, S.L.
(86) International application number: PCT/ES2005/000046
(87) International publication number: WO 2005/076000

(56) References cited:
- EP-A1- 0 600 088
- US-A- 6 037 450
- US-B2- 6 617 145
- OOSTING J.D. ET AL: 'Antiphospholipid antibodies directed against a combination of phospholipids with prothrombin, protein C, or protein S: an explanation for their pathogenic mechanism?.' BLOOD. vol. 81, no. 10, May 1993, pages 2618 - 2625, XP008070727
- GU J.M. ET AL: 'Disruption of the endothelial cell protein C receptor gene in mice causes placental thrombosis and early embryonic lethality.' THE JOURNAL OF BIOLOGICAL CHEMISTRY. vol. 277, no. 45, November 2002, pages 43335 - 43343, XP008042848

## Description

### FIELD OF THE INVENTION

The present invention is related to a method to detect elevated levels of autoantibodies against endothelial protein C / activated protein C receptor (EPCR) in a sample, by its detection and *in vitro* quantification.

### BACKGROUND OF THE INVENTION

### Autoimmune diseases

Autoimmune diseases are characterized by the presence of immune reactions in which some factor induces an immune reaction against the host tissues, and the production of abnormal antibodies that attack such tissues (autoantibodies). These autoimmune diseases include disorders such as antiphospholipid syndrome (APLS), rheumatoid arthritis, systemic lupus erythematosus, autoimmune vasculitis in general, etc.

APLS is characterized by vascular thrombosis (venous, arterial or microvascular) and complications during pregnancy (fetal death, premature birth or multiple spontaneous miscarriage) associated with the presence of antiphospholipid antibodies. These antibodies are heterogeneous and recognize a variety of combinations of phospholipids, phospholipid binding proteins, or both. The most commonly detected antiphospholipid antibody subgroups comprise the so-called anticoagulant lupus antibodies (ACL), anticardiolipin antibodies and antiglycoprotein I β2 antibodies. Other antiphospholipid antibodies not included in the classical laboratory criteria are presently being investigated. Such antibodies are targeted to phospholipids other than cardiolipin, such as phosphatidylethanolamine, or to phospholipid binding proteins such as annexin V and protein S. However, little is known about the mechanisms relating the presence of antiphospholipid antibodies to vascular thrombosis and miscarriage.

### Vascular diseases

Vascular diseases are of three principal types, depending on the kind of vessel involved (arterial, venous or small-caliber vessels of the microcirculation). In the case of arterial vascular diseases, parietal sclerosis reduces blood flow through the vessel lumen, and therefore chronically reduces blood supply to the territories irrigated by the damaged blood vessel. This atherosclerotic lesion can suffer complications and give rise to thrombus formation within the artery - totally occluding the latter and thus obstructing blood flow entirely. In this case tissue infarction results. The most frequent examples of this phenomenon are myocardial infarction, when thrombosis affects a coronary artery, or stroke - when the affected vessel is a brain artery. In the case of venous vascular disease, thrombosis complicates return blood flow to the heart. When a fragment of the thrombus in the thrombotic venous wall becomes detached, it will migrate within the bloodstream until it becomes lodged in the pulmonary venous circulatory circuit - giving rise to acute pulmonary failure (a situation known as pulmonary embolism). Diseases of the microcirculation develop secondary to inflammation and/or thrombosis of the vessels of the microcirculation in different organs, and manifest as organ failure. Vascular diseases are an important cause of morbidity and mortality in western countries. Specifically, according to data from the *Instituto Nacional de Estadística* (INE) (Spanish National Institute of Statistics) corresponding to the year 2000, cardiovascular diseases are the first cause of death in Spain (representing approximately 35.0% of total mortality). Among the most frequent cardiovascular disorders, vascular or thrombotic arterial diseases of the heart (mainly acute myocardial infarction) constitute the first cause of death. At present, a number of molecular risk factors have been identified that can account for the appearance of thrombosis in some patients. One such risk factor is the presence of so-called antiphospholipid antibodies. It was originally believed that these autoantibodies were targeted to anionic phospholipids; however, posteriorly it has been shown that many of these autoantibodies are targeted to complexes formed between proteins such as glycoprotein I β2 or prothrombin, and phospholipids. More recently, other proteins with anticoagulant roles have also been implicated, such as protein C (PC), protein S, thrombomodulin or annexin V - thus explaining why the presence of these autoantibodies predisposes to thrombosis.

### Obstetric complications

Obstetric complications fundamentally comprise fetal death after the tenth week of pregnancy, the birth of premature infants, spontaneous miscarriage before the tenth week of pregnancy, delayed intrauterine growth, eclampsia and pre-eclampsia.

### EPCR

Activated protein C (APC) is one of the principal coagulation cascade regulatory proteins. PC, the zymogen of APC, is activated by thrombin bound to thrombomodulin on the surface of the endothelial cells. APC, in combination with protein S (its non-enzymatic cofactor), exerts its anticoagulant role via the proteolysis of activated factors V and VIII. Genetic and acquired defects in thrombomodulin, PC and protein S have been detected in patients with venous and/or arterial thrombosis. Endothelial PC / activated PC receptor (EPCR) is a glycoprotein expressed on the membrane of endothelial cells that specifically and with high affinity binds PC and APC. In order for EPCR to be functional, it must be bound to a phospholipid molecule that stabilizes its three-dimensional structure. The binding of PC to EPCR markedly increases its activation by the thrombin-thrombomodulin complex on the endothelial cell surface. The mission of EPCR is to concentrate PC on the endothelial surface and present it to the thrombin-thrombomodulin complex - thereby favoring efficient PC activation. EPCR induces an approximately 9-fold increase in the PC activation index on the surface of endothelial cells *in vivo -* as a result of which it is responsible for 90% of the circulating levels of APC. Moreover, only when APC is bound to EPCR can it activate protease-activated receptor-1, that generates a "cytoprotective" cell signal and blocks apoptosis.

EPCR is mainly expressed by the endothelium of veins and arteries, particularly those of large and medium caliber. Moreover, it is intensely expressed by the syncytiotrophoblast. In these locations EPCR prevents thrombosis and favors good cell function both of the endothelium and the syncytiotrophoblast. There is increasingly solid evidence to suggest that EPCR plays a role in the maintenance of pregnancy, since deletion of the EPCR gene in knock out mice causes placental thrombosis and early embryonic death in these mice.

US6037450 describes a method for the diagnosis of inflammation and disease states associated with abnormal coagulation based on the determination of the levels of plasma EPCR.

### COMPENDIUM OF THE INVENTION

The present invention refers to a method for the determination of anti-EPCR autoantibodies (IgG, IgA and IgM) in a sample from a subject. On the other hand, it has been demonstrated that these autoantibodies are present in patients diagnosed with autoimmune diseases (APLS and disseminated lupus erythematosus), in patients with vascular diseases (venous and arterial thrombosis) and in female patients with obstetric complications. The examples that accompany the present description illustrate, among other things, the fact that the presence of anti-EPCR autoantibodies in serum or plasma is increased in patients with autoimmune diseases (determined in patients with APLS or disseminated lupus erythematosus), in patients with vascular diseases, such as arterial thrombosis, for example, myocardium infarction (determined both in patients with APLS and in those without), or ischemic stroke (determined in patients with APLS), or venous thrombosis (determined in patients with APLS), as well as in patients with obstetric complications, such as fetal death (determined both in women with APLS and in women without APLS), or multiple miscarriage (determined in patients with APLS).

The authors of the present invention have discovered that the presence of anti-EPCR autoantibodies in serum or plasma from patients with autoimmune diseases, and/or patients with vascular diseases and/or in patients with obstetric complications, is increased when compared with samples from healthy subjects, not affected by such diseases. This evidence makes said anti-EPCR autoantibodies a useful marker for *in vitro* evaluation of the risk and susceptibility of a subject to develop a disease associated with the presence of increased levels of autoantibodies against EPCR, such as an autoimmune disease, a vascular disease, or obstetric complications.

Studies have been made of the presence of anti-EPCR autoantibodies in patients with APLS and their relation to fetal death. An evaluation has also been made of the effect of these autoantibodies upon the generation of APC on the endothelial surface. Afterwards, a study has been made of the association of anti-EPCR autoantibodies to fetal death in a paired case-control study. The results obtained support the notion that anti-EPCR autoantibodies constitute a risk factor for fetal death. Prevention of the activation of PC on the cell surfaces that express EPCR could be one mechanism by which these autoantibodies exert their pathological effects.

### BRIEF DESCRIPTION OF THE FIGURES

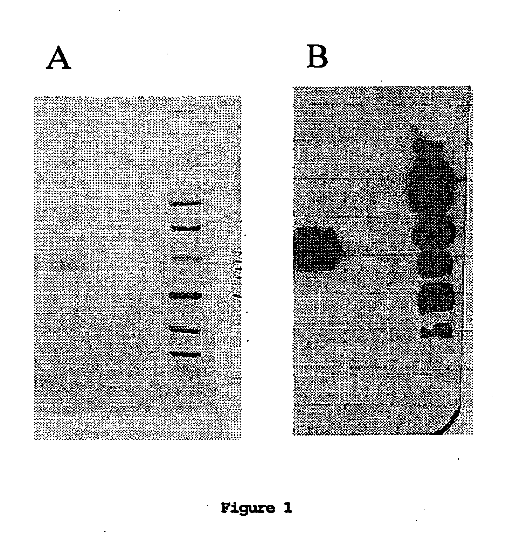

Figure 1 shows the expression of rhsEPCR *in Pichia pastoris.* The rhsEPCR was purified from the supernatant of stably transformed *P. pastoris* cells, as described in the section relating to Materials and Methods (see Example). Ten µl of each of three fractions containing rhsEPCR were separated by SDS-PAGE, and the proteins were detected using GELCODE Blue (A) or via Western blot with the monoclonal anti-myc antibody (Invitrogen)(B).

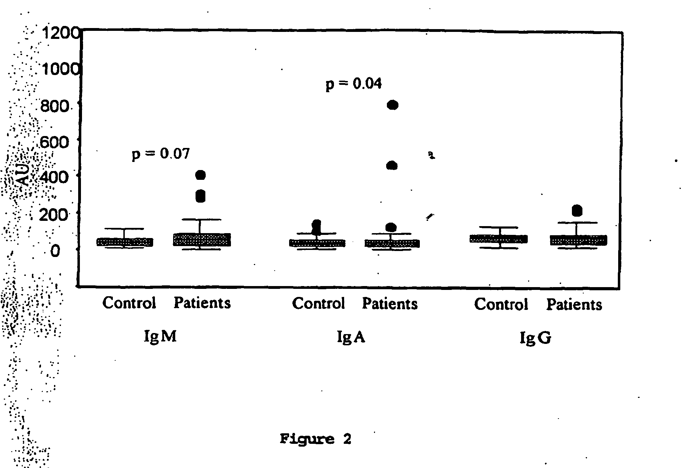

Figure 2 compares the level of anti-EPCR autoantibodies in patients diagnosed with APLS and in controls. The levels of anti-EPCR autoantibodies are shown. Antibodies of IgM isotype: controls (median = 45 AU, arbitrary units), patients (median = 57 AU); antibodies of IgA isotype: controls (median = 31 AU), patients (median = 39 AU); and antibodies of IgG isotype: controls (median = 72 AU), patients (median = 75 AU).

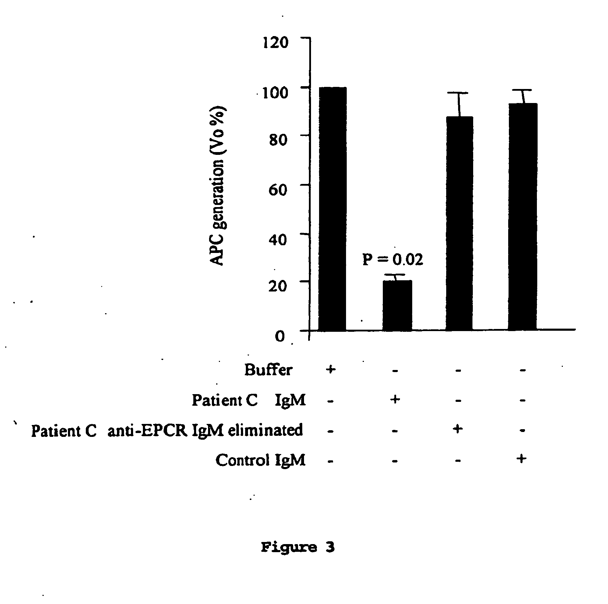

Figure 3 shows the effect of anti-EPCR autoantibodies on the generation of APC by endothelial cells, where the generation of APC in the presence of anti-EPCR autoantibody of isotype M can be seen in patient C, compared with the generation of APC in the absence of antibody and in the presence of non-inhibiting antibody. For each condition 2-4 independent experiments were performed.

### DETAILED DESCRIPTION OF THE INVENTION

### Definitions

In order to facilitate understanding of the present patent application, the meanings of some terms and expressions used in the context of the invention are explained below.

The term "subject" refers to a member of a mammalian species, and includes but is not limited to domestic pets, primates and humans; the subject is preferentially a human (male or female) of any age or race. The term "individual" is used indifferently.

The expression "autoimmune diseases" refers to those disorders in which the immune system reacts against the host tissues, giving rise to a broad range of disorders. For illustrative purposes, such diseases include (among other conditions) APLS, systemic lupus erythematosus, rheumatoid arthritis, autoimmune vasculitis, etc.

The expression "vascular diseases" refers to those disorders that affect the blood vessels. When an artery is involved, the subsidiary territory irrigated by the vessel suffers a lack of perfusion; this condition is usually secondary to arterial occlusion attributable to an atherosclerotic lesion in the wall or to thrombosis or both simultaneously. Venous involvement is in turn defined by the complication of blood return to the heart from the affected peripheral territory, and is usually the result of venous thrombus formation leading to vessel occlusion. Involvement of the microcirculation is characterized by functional impairment of the affected organ. As an example, such diseases include (among other disorders) arterial vascular disorders such as myocardial infarction, stroke, transient cerebrovascular accidents, ischemia of the limbs, atherosclerosis, aneurysms, etc., as well as venous vascular diseases such as superficial and deep venous thrombosis, pulmonary embolism, etc., and microcirculatory pathology (thrombosis) in the form of organ failure seen during infections or in the context of autoimmune diseases.

The expression "obstetric complications" refers to those disorders affecting the development of pregnancy, as relates to both the gestating mother and to embryo or fetus. Examples include miscarriage, fetal death, premature birth, delayed intrauterine growth, eclampsia and pre-eclampsia.

The term "autoantibody" refers to the antibodies produced by an individual and targeted to (or specific for) host structures and tissues. Examples include antiplatelet autoantibodies, antithyroid autoantibodies, autoantibodies targeted to erythrocytes, etc. In this sense, the term "anti-EPCR autoantibody" refers to immunoglobulins or antibodies produced by the subject and specifically targeted to EPCR of his or her own tissues.

The term "epitope", as used in the present invention, refers to an antigenic determinant of a protein, such as the amino acid sequence of the latter, which is recognized by the antibody in question.

The terms "peptide" and "polypeptide" refer to molecular chains of amino acids that represent a protein fragment. The terms "protein" and "peptide" are used indistinctly.

The present invention is based on the observation that the production of anti-EPCR autoantibodies in patients with autoimmune diseases, and/or in patients with vascular diseases and/or in patients with obstetric complications is increased in comparison to samples from healthy individuals without such diseases. This evidence defines such anti-EPCR autoantibodies as a useful marker for *in vitro* evaluation of the risk and susceptibility of a given individual to develop a pathology related to the presence of high levels of autoantibodies against EPCR.

As used in this description, the expression "high levels of anti-EPCR autoantibodies" refers to levels of AU (arbitrary units) equal to or in excess of percentile 50 in the normal population, including, for example, levels of AU equal to or in excess of percentile 60 in the normal population, equal to or in excess of percentile 70 in the normal population, equal to or in excess of percentile 80 in the normal population, equal to or in excess of percentile 90 in the normal population, and equal to or in excess of percentile 95 in the normal population. Due to inter-individual variability (e.g., aspects relating to race, etc.) it is very difficult (if not practically impossible) to establish absolute values indicative of high levels of anti-EPCR autoantibodies applicable to all individuals. Such percentiles can easily be calculated by means of a conventional procedure involving the testing of a group of normal subjects (i.e., people with no diagnosis of autoimmune disease, or antecedents of vascular disease or obstetric complication at the time of testing) of the levels of anti-EPCR autoantibodies. The determination of anti-EPCR autoantibodies can be done using any conventional method, for example the ELISA described under "Materials and Methods" (Example 1). Logically, each subject will present a certain level (AU) of anti-EPCR autoantibodies, and a concrete anti-EPCR autoantibody level will be identified above which 50% of the analyzed population is found. This value is the percentile 50. Obviously, a value (AU) also exists above which 40% of the normal subjects tested can be found - this value corresponding to percentile 60. In turn, other values can be defined above which 30%, 20%, 10% and 5% of the normal subjects tested can be found - corresponding to percentiles 70, 80, 90 and 95, respectively.

The invention provides a method to evaluate the risk and susceptibility of an individual to develop a pathology related to the presence of high levels of autoantibodies against EPCR, comprising the *in vitro* quantification of autoantibodies against EPCR in a sample of the mentioned subject.

In a particular elaboration, the pathology related to the presence of high levels of autoantibodies against EPCR in an individual is selected from among an autoimmune disease, for example, APLS, systemic lupus erythematosus, rheumatoid arthritis, autoimmune vasculitis, etc.; a vascular disease, for example, arterial vascular disease such as myocardial infarction, stroke, transient cerebrovascular accidents, ischemia of the limbs, atherosclerosis, aneurysms, thrombosis, etc., or venous vascular disease such as superficial or deep venous thrombosis, pulmonary embolism, etc., or microcirculatory vascular disease; and obstetric complications, for example, miscarriage, fetal death, premature birth, delayed intrauterine growth, eclampsia, pre-eclampsia, etc.

The method provided by the present invention is applicable to the determination of the variation of the levels of anti-EPCR autoantibodies over a given timespan. Such determinations object of the present invention are completed by their comparison to normal levels of anti-EPCR autoantibodies.

This method comprises a step in which a sample is collected from the individual, such as a sample of serum or plasma, which can be obtained by any conventional method, e.g., blood collection.

The samples can be obtained from individuals with previously diagnosed or non-diagnosed autoimmune diseases or vascular disorders, or obstetric complications. They can also be obtained from individuals undergoing treatment, or that have been previously treated for such diseases or complications.

Given the nature of the method of the invention, the detection and quantification of these anti-EPCR autoantibodies is carried out by means of an immune test coupled to a marker that allows detection and quantification of the formation of specific antigen-antibody complexes, e.g., an immunochromatographic test (latex, colloidal gold, etc.), an immune test in which the marker is fluorescent, an isotope, a heavy metal, an enzyme, a luminescent marker, a chemiluminescent marker, a chromogen, etc.

A broad range of well known tests can be used in the present invention, involving the use of unlabeled antibodies (primary antibody) and labeled antibodies (secondary antibody). These techniques include Western-blot or Western transference, ELISA (enzyme-linked immunosorbent assay), RIA (radioimmunoassay), etc.

In a particular elaboration, the preferred immune test in the method of the invention, which allows the detection and/or quantification of these anti-EPCR autoantibodies is an ELISA test comprising the following aspects:
a) Solid support immobilization of a polypeptide comprising the sequence of amino acids of EPCR or a fragment of the latter containing at least one epitope that can be recognized by an anti-EPCR autoantibody;
b) Incubation of the immobilized polypeptide with a sample suspected to contain anti-EPCR autoantibodies, obtained from the subject, for sufficient time to allow binding of the antibodies to the immobilized polypeptide, and the formation of polypeptide-anti-EPCR autoantibody complexes;
c) Removal of remaining sample not bound to the immobilized polypeptide;
d) Incubation of the polypeptide-anti-EPCR autoantibody complexes with a second antibody conjugated to an enzyme, where the second antibody is able to bind to these anti-EPCR autoantibodies.

This polypeptide comprising the EPCR amino acid sequence or a fragment of the latter containing at least one epitope that can be recognized by an anti-EPCR autoantibody can be a polypeptide comprising the sequence of amino acids of full length EPCR, or a polypeptide comprising the sequence of amino acids of an EPCR fragment and containing at least one epitope capable of being recognized by an anti-EPCR antibody. In a particular elaboration, the mentioned polypeptide is a fusion protein comprising:
(i) a region A composed of a polypeptide containing the EPCR amino acid sequence or a fragment of the latter containing at least one epitope capable of being recognized by an anti-EPCR antibody; and
(ii) a region B composed of a polypeptide containing an amino acid sequence of use for isolating or purifying the mentioned fusion protein, and/or a sequence of amino acids of use for anchoring the mentioned fusion protein to a solid support.

This region B can be bound to the amino terminal extreme of region A or to the carboxyl terminal extreme of region A.

In a particular elaboration, region A comprises the amino acid sequence of the soluble part of human EPCR.

Region B comprises an amino acid sequence of use for the isolation or purification of the previously defined fusion protein, and/or a sequence of amino acids of use for anchoring the mentioned fusion protein to a solid support. Practically any sequence of amino acids that can be used to isolate or purify a fusion protein (generically referred to as "tag" peptides), and/or any sequence of amino acids capable of being used for anchoring a fusion protein to a solid support can be present in region B. Occasionally, the amino acid sequence of use for the isolation or purification of the fusion protein can also act as a sequence of amino acids of use for anchoring the mentioned fusion protein to a solid support, and vice versa. In a particular elaboration, region B comprises a sequence of amino acids of use for the isolation or purification of a fusion protein and a sequence of amino acids of use for anchoring a fusion protein to a solid support.

As an example, this sequence of amino acids of use in isolating or purifying a fusion protein and/or the sequence of amino acids of use in anchoring a fusion protein to a solid support can be Arg-tag, His-tag, FLAG-tag, Strep-tag, an epitope capable of being recognized by an antibody, such as c-myc-tag, SBP-tag, S-tag, calmodulin binding peptide, cellulose binding domain, chitin binding domain, glutathione S-transferase-tag, maltose binding protein, NusA, TrxA, DsbA, Avi-tag, etc. (Terpe K., Appl. Microbiol. Biotechnol. (2003), 60:523-525), a sequence of amino acids such as Ala-His-Gly-His-Arg-Pro (SEQ ID NO: 4) (2, 4, and 8 copies), Pro-Ile-His-Asp-His-Asp-His-Pro-His-Leu-Val-Ile-His-Ser (SEQ ID NO: 5), Gly-Met-Thr-Cys-X-X-Cys (SEQ ID NO: 6) (6 repetitions), β-galactosidase, VSV-glycoprotein (YTDIEMNRLGK), etc.

In a particular elaboration, region B is composed of a polypeptide comprising an epitope capable of being recognized by an antibody (such as epitope c-myc, recognized by an anti-c-myc antibody), and a tail of histidines (His-tag).

In the Example accompanying this description, an account is provided of the obtention of a polypeptide, called rhsEPCR, consisting of a fusion protein comprising the amino acid sequence of the soluble part of human EPCR (hsEPCR), the amino acid sequence corresponding to c-myc epitope and a tail of histidines the amino acid sequence being shown in SEQ ID NO: 3.

The polypeptide to be used in the method of the invention can be obtained by conventional methods, for example, by expression in an appropriate expression system.

The second antibody to be used in the previously mentioned ELISA test is an immunoglobulin isotype-specific antibody originating from a species different to that of the study subject, thereby allowing characterization of the isotype of the anti-EPCR autoantibodies. As an example, this second antibody specific of a given immunoglobulin isotype is selected from among an anti-human IgG antibody, an anti-human IgM antibody, an anti-human IgA antibody, and their mixtures. In a particular elaboration, the second antibody is conjugated to a marker allowing detection of the complex, such as an enzyme (e.g., peroxidase, alkaline phosphatase, etc).

In another aspect, the invention supplies a method to evaluate the risk and susceptibility of an individual to develop a pathology related to the presence of high levels of anti-EPCR autoantibodies in said individual, comprising *in vitro* quantification of autoantibodies against EPCR in a sample from said individual.

In a particular embodiment, the pathology related to the presence of high levels of autoantibodies against EPCR in an individual is selected from among an autoimmune disease, such as APLS, systemic lupus erythemotosus, reumatoid arthritis, autoimmune vasculitis, etc.; a vascular disease, such as, an arterial vascular disease, e.g. myocardial infarction, stroke, transient cerebrovascular accidents, ischemia of limbs, atherosclerosis, aneurysms, thrombosis, etc., or a venous vascular disease, e.g. superficial o deep venous thrombosis, pulmonary embolism, etc. or microcirculatory vascular disease such as a microcirculatory thrombosis, organic failure occurring during infections or autoimmune disease, etc.; and an obstetric complication, for example, miscarriage, fetal death, premature birth, delayed intrauterine growth, eclampsia, pre-eclampsia, etc.

The method provided by the present invention is based on the fact that individuals diagnosed with an autoimmune or vascular disease o with obstetric complications, have high levels of anti-EPCR autoantibodies, when compared to the corresponding levels in individuals without a clinical history of such diseases or obstetric complications.

The method used to evaluate the risk and susceptibility of an individual to develop a pathology related to the presence of high levels of anti-EPCR autoantibodies provided by this invention is completed by comparing the levels of autoantibodies determined in the study subject sample with normal levels (defined as those found in a population of normal individuals such as that mentioned above in reference to definition of the expression "high levels"). Said method is based on immunological assays previously described in this section.

In another aspect, the invention is related to a method for *in vitro* monitorization of the effect of therapy administered to an individual presenting a pathology related to the presence of high levels of anti-EPCR autoantibodies, comprising the *in vitro* quantification of these anti-EPCR autoantibodies in a sample of the mentioned subject. The method is carried out as mentioned above, though in this case the samples originate from subjects previously diagnosed with some autoimmune or vascular disease, or presenting some obstetric complication, subjected to therapy. The method allows evaluation of the effect of therapy, i.e., its efficacy and effectiveness, applied to the subject undergoing treatment, with the purpose (for example) of either maintaining therapy or modifying it.

In another aspect, the invention is related to the use of anti-EPCR autoantibodies in a method to evaluate the presence of high levels of autoantibodies against EPCR in a sample from an individual. In a particular embodiment, said presence of high levels of anti-EPCR autoantibodies related to a pathology, is selected from among an autoimmune disease, a vascular disease and obstetric complications. An increased level of anti-EPCR autoantibodies in the subject is associated with an increased risk or susceptibility to develop a pathology related with the presence of high levels of anti-EPCR autoantibodies, such as autoimmune disease, vascular disease and/or obstetric complications.

In another aspect, the invention is related to the use of a polypeptide comprising the sequence of amino acids of EPCR or a fragment of the latter containing at least one epitope capable of being recognized by an anti-EPCR autoantibody, in a method for evaluating the presence of autoantibodies against the endothelial receptor EPCR in a sample. In a particular elaboration, this pathology related to the presence of high levels of anti-EPCR autoantibodies was selected from among an autoimmune disease, a vascular disease and obstetric complications.

In a particular elaboration, the mentioned polypeptide comprising the EPCR amino acid sequence or a fragment of the latter containing at least one epitope capable of being recognized by an anti-EPCR autoantibody is a polypeptide such as that previously defined on describing the ELISA test for detecting and/or quantifying anti-EPCR autoantibodies. In a particular elaboration, this polypeptide is the so-called rhsEPCR (see Example), consisting of a fusion protein comprising the sequence of amino acids of the soluble part of human EPCR (haEPCR), the amino acid sequence corresponding to c-myc epitope and a tail of histidines - the sequence of which is shown in SEQ ID NO: 3.

In another aspect, the invention is related with a kit designed for *in vitro* evaluation of high levels of anti-EPCR autoantibodies, comprising a polypeptide with the EPCR amino acid sequence or a fragment of the latter containing at least one epitope capable of being recognized by an anti-EPCR autoantibody and an antibody specific of a given immunoglobulin isotype, wherein said antibody is selected from an anti-human IgG antibody, and anti-human IgM antibody and an anti-human IgA antibody and their mixtures. In a particular elaboration, this polypeptide comprising the EPCR amino acid sequence or a fragment of the latter containing at least one epitope capable of being recognized by an anti-EPCR autoantibody, is a polypeptide such as that previously defined on describing the ELISA test for detecting and/or quantifying anti-EPCR autoantibodies. In a particular elaboration, this polypeptide is the so-called rhsEPCR (see Example), consisting of a fusion protein comprising the amino acid sequence of the soluble part of human EPCR (hsEPCR), the amino acid sequence corresponding to c-myc epitope, and a tail of histidines
- the sequence of which is shown in SEQ ID NO: 3.

In another aspect, said kit is used to evaluate *in vitro* the risk and susceptibility of an individual to develop a pathology associated with the presence of high levels of anti-EPCR autoantibodies, selected from among an autoimmune disease, a vascular disease and obstetric complications.

The following examples illustrate the invention.

### EXAMPLE 1

### Use of anti-EPCR autoantibodies as markers for the risk and susceptibility of an individual to develop pathologies related to the presence of high levels of these autoantibodies

### I. MATERIALS AND METHODS

### Patients

### 1. Patients with APLS and controls

The study comprised a total of 43 patients [age 44 ± 11 years (mean ± standard deviation (SD)), 39 women and 4 males] diagnosed with antiphospholipid syndrome (APLS) according to international diagnostic criteria [Wilson WA, Gharavi AE, Koike T, Lockshin MD, Branch DW, Piette JC, Brey R, Derksen R, Harris EN, Hughes GR, Triplett DA, Khamashta MA. International consensus statement on preliminary classification criteria for definite antiphospholipid syndrome: report of an international workshop. Arthritis Rheum. 1999;42:1309-11; Brandt JT, Barna LK, Triplett DA. Laboratory identification of lupus anticoagulants: results of the Second International Workshop for Identification of Lupus Anticoagulants. On behalf of the Subcommittee on Lupus Anticoagulants / Antiphospholipid Antibodies of the ISTH. Thromb Haemost. 1995;74:1597-603] between February 1998 and March 2002. All patients were characterized by presenting anticoagulant lupus antibodies (ACL) and a personal history of venous thrombosis (n = 17), arterial thrombosis (n = 13, of which 4 referred acute myocardial infarction (AMI), 7 presented cerebrovascular thrombotic disease (CVTD), and 2 showed disease in other regions), or both [n = 13, all presenting deep venous thrombosis plus CVTD (n = 8), AMI (n = 1), CVTD plus AMI (n = 3) or arterial thrombosis in the mesenteric region (n = 1)]. Twenty-seven of these patients were diagnosed with systemic lupus erythematosus (SLE). Serum samples were collected during the time in which ACL was positive, and at least 3 months after the last thrombotic episode. The samples were stored at -80°C until processing for the detection of anti-EPCR autoantibodies.

The control group consisted of 43 healthy volunteers with no history of thrombosis or ACL. All patients and controls had given informed consent to participation in the study.

### 2. Women with fetal death and controls

A paired case-control study of fetal death was carried out. A total of 87 women, aged 19 to 31 years (mean: 27 years), were included in the study between September 1996 and September 2002 due to a first episode of fetal death in the tenth week of amenorrhea, and occurring in their last pregnancy. The study excluded women with thrombotic antecedents, a history of chronic infectious diseases or some known systemic disease, diabetes mellitus or with antecedents of other types of gestational pathology (spontaneous miscarriage, eclampsia, restricted intrauterine fetal growth), as well as cases of fetal death due to some chromosomal anomaly affecting the karyotype, or morphological malformation of the fetus. Fetal death occurred during first pregnancy in 58 women, during second pregnancy in 21 women, and during third pregnancy in the remaining 8 women; in 75 women the event took place between weeks 10 and 22, while in the remaining 12 women fetal death occurred between weeks 22 and 36 (mean: 17 weeks).

A control group of 87 healthy mothers was established, grouped by age, number of pregnancies and the time elapsed from the last pregnancy; all satisfied the exclusion criteria applied to the group of women with fetal death. The controls were recruited concomitantly during the same time period, from among women seen as outpatients in the Department of Gynecology of the same Hospital, for systematic medical examination.

The study was approved by the ethics committees of the inventors institution, and informed consent was obtained from all subjects. The inclusion of patients and controls, informed consent, and the collection of blood samples took place at least 6 months (range: 6-12 months) after fetal death. The blood samples were collected, processed and stored at -80°C, according to conventional procedures. The sampling protocols were identical in all cases and controls.

### Expression of recombinant human soluble EPCR

For the expression of human recombinant EPCR in soluble form (rhsEPCR), amplification was performed of the human soluble EPCR (hsEPCR) sequence, comprising the extracellular domain without its signal peptide or the transmembrane and intracellular domains (Entrez-Protein 21730830, residues 1-193, numbering corresponding to the mature form of the protein after processing of the signal peptide), via polymerase chain reaction (PCR) with the primers
SEQ ID NO: 1 and
SEQ ID NO: 2,
which added a ClaI restriction site and another NotI site at the 5' and 3' extremes, respectively, using cDNA from endothelial cells as template. These modifications allowed binding of the rhsEPCR sequence to the ClaI and NotI sites of plasmid pPICZαC (Stratagene, La Jolla, CA) following the secretion signal of factor a from *Saccharomyces cerevisiae,* permitting efficient secretion of many proteins into the extracellular medium from the interior of yeast cells.

The insert was cloned in reading phase with a c-myc epitope and a 6-histidine tag present in the pPICZaC vector. Due to the cloning process a serine residue and an isoleucine residue were added at the amino extreme of rhsEPCR, which is expressed fused to its carboxy-terminal end to a tail or tag containing the c-myc epitope and 6 histidines to facilitate purification and anchoring of rhsEPCR to the bottom of the microplate wells via an anti-c-myc monoclonal antibody. By direct sequencing, it was confirmed that the insert and vector sequences were correct. SEQ ID NO: 3 shows the sequence of rhsEPCR thus obtained, deduced from the DNA sequence, comprising the residues added by the cloning technique employed, the residues of the extracellular region of human rhsEPCR, the c-myc epitope, and the tag of 6 histidines.

With the previously prepared expression vector and after linearization of the latter with restriction enzyme PmeI, *Pichia pastoris* cells were transformed by means of a chemical method (Easy Comp, Invitrogen), yielding the integration, via homologous recombination, of the sequence encoding rhsEPCR in the methanol response endogenous promoter. The transformation product was cultured in presence of zeocine to select those colonies of *P. pastoris* transformed with the vector containing the rhsEPCR encoding sequence, which in turn contains the gene encoding resistance to zeocine. Briefly, the transformed yeasts were cultured in 4 ml of BMY medium [1% (w/v) of yeast extract, 2% (w/v) of peptone, potassium phosphate 100 mM (pH 6.0), 1.34% (w/v) of yeast nitrogen source with ammonium sulfate, 4x10-5% (w/v) of biotin] supplemented with 1% (v/v) of glycerol (BMGY), and incubated at 28-30°C for about 18 hours with stirring. Cells were collected by centrifugation at 2000 g during 5 minutes at room temperature. The supernatant was discarded, and expression of rhsEPCR was induced with 1% methanol during 18 hours. To this effect, the cells were resuspended in 3 ml of BMY supplemented with 0.5% (w/v) of methanol, followed by incubation for 18 hours at approximately 28-30°C under vigorous stirring. Following induction, the samples from the conditioned medium were loaded on 12% NuPAGE Bis-Tris gels (Invitrogen, Carlsbad, CA), and rhsEPCR was detected by Western Blot using the anti-myc monoclonal antibody (Invitrogen). For large scale production, selection was made of the colony that secreted the highest concentration of rhsEPCR. From the colonies selected on the basis of their high production of rhsEPCR, studies were made of colony methanol metabolism (rapid or slow metabolizer), thus allowing definition of the optimum expression conditions for the most adequate colony. Following optimization of the culture conditions and induction with methanol, the scale was increased for the production of large amounts of rhsEPCR.

### Purification of recombinant sEPCR

Since *P. pastoris* secretes very few proteins into the medium, a high percentage of the proteins found in the culture medium correspond to rhsEPCR - a fact that considerably simplified purification of the latter. Briefly, rhsEPCR was purified from the supernatants of the yeast cultures by means of a triple-step purification process comprising metal affinity chromatography, anion exchange and gel filtration chromatography. To this effect, the supernatant of the culture was concentrated and dialyzed against sodium phosphate 100 mM, NaCl 10 mM, pH 7.6, followed by metal affinity chromatography in a 5-ml Hitrap column (Amersham Biosciences, Little Chalfont, United Kingdom) loaded with copper. The fraction that bound to the column was eluted with a buffer containing ethylenediaminetetraacetic acid (EDTA), and was dialyzed against Tris-HCl 20 mM (pH 7.6) without NaCl. Next, anion exchange chromatography was carried out in a Resource Q column (Amersham Biosciences), and elution was performed with a 0.0-300 mM gradient of NaCl in a volume equivalent to that of 20 columns. The eluted fractions containing rhsEPCR were pooled and concentrated by centrifugation-ultrafiltration, and then loaded on a Superdex 75-HR10/30 column (Amersham Biosciences) for gel filtration. The concentration of purified protein was determined using the BCA total protein test (Pierre, Rockford, IL) and standards of bovine serum albumin (BSA). For the detection of purified rhsEPCR, the samples were loaded on 12% NuPAGE Bis-Tris gels (Invitrogen, Carlsbad, CA), and electrophoresis was performed under reducing conditions followed by staining with Coomassie blue. One electrophoresis gel was subjected to electroblotting, and rhsEPCR was detected with anti-myc monoclonal antibody (Invitrogen). In order to estimate the molecular weight of rhsEPCR, use was made of a molecular weight standard included in each electrophoresis gel.

### ELISA for the determination of anti-EPCR autoantibodies in serum or plasma

Separate determinations were made of the levels of anti-EPCR autoantibodies corresponding to isotypes IgG, IgA or IgM, since these are the forms most frequently found in patients with autoimmune alterations, where antibodies targeted to some of the host structures are detected (autoantibodies).

In all three cases 96-well microplates (Costar, Acton, MA, USA) were coated with 100 µl/well of anti-c-myc monoclonal antibody (Invitrogen, USA) at a concentration of 1.5 µg/ml in a solution of Na₂CO₃ (100 mmol/l), pH 9.6, overnight at a temperature of 4°C. This antibody is used as capture antibody, and is targeted to the added c-myc tag present in rhsEPCR. In this way, rhsEPCR is anchored to the well, preserving its extracellular epitopes. After washing with TB (Tris 20 mM, NaCl 150 mM, 0.05% of Tween-20, pH 7.4), the nonspecific binding sites were blocked with 3% (w/v) of BSA in TB at room temperature (RT) during 4.5 hours. Then, 100 µl/well of a solution containing 3 µg/ml of rhsEPCR in TB supplemented with 1% BSA (TB1) was added, followed by incubation during 2 hours at room temperature with gentle stirring. In parallel, blank wells were incubated with TB1 in the absence of rhsEPCR. After washing with TB, 100 µl of a 1:100 (plasma or serum) dilution of the sample in TB1 was added to each well, followed by incubation overnight at 4°C. The wells were then washed with TB, and the anti-EPCR autoantibodies remaining bound to the bottom of the wells were detected with murine anti-human IgA polyclonal antibody conjugated to peroxidase (Biotrend), murine anti-human IgM polyclonal antibody conjugated to peroxidase (Zymed), or murine anti-human IgG polyclonal antibody conjugated to alkaline phosphatase (Zymed). After a 2 hour incubation period at room temperature with gentle stirring, washing was performed.

To determine the levels of anti-EPCR autoantibodies of IgA or IgM isotype, 100 µl of a solution (0.4 mg/ml) of or-phenylendiamine (Kodak) was added, containing Na₂HPO₄ 0.07 M, sodium citrate 0.04 M and 0.02 % (v/v) of H₂O₂, pH 5.0. After a development period of 5 and 8 minutes in the dark for IgA and IgM, respectively, 100 µl of H₂SO₄ was added to stop the reaction, and 5 minutes later readings were obtained of the absorbances at 492 nm in a microplate reader (iEMS REader, Labsystems, Finland).

In the plate used to assay anti-EPCR autoantibodies of IgG isotype, 100 µl of a solution (1 ng/ml) of 4-nitrophenyl phosphatase (Sigma) in diethanolamine 0.1 M, pH 10.3 was added. After 15 minutes, the reaction was stopped with 100 µl of NaOH 1 M, and the absorbance was recorded after color stabilization at 405 nm in the microplate reader (iEMS REader). All samples were assayed at least twice in different tests.

To ensure that all absorbances measured in each plate corresponded to a linear range, construction was made, for each isotype, of a curve using serial dilutions of the sample whose absorbance was the highest recorded. To allow comparisons between plates, a sample was selected for testing in each plate (standard sample) - thus allowing introduction of a correction factor. The arbitrary units (AU) were defined as follows: for each patient sample (study sample) the specific absorbance was calculated substracting the absorbance of the blank wells and then multiplying by 1000 and by a correction factor corresponding to the ratio between the specific absorbance of the standard sample tested in a given plate (reference plate) and in the plate where the study sample was tested. The inter- and intra-test coefficients of variation (CV) were evaluated using 5 samples tested 5 times for the inter-test coefficient of variation (less than 5%), and three different times for calculating the inter-test coefficient of variation (less than 10%).

### Generation of APC in cultured endothelial cells

The cell line used was EA.hy926, a line of transformed human endothelial cells that have retained the capacity to express thrombomodulin and EPCR (Stearns-Kurosawa DJ, Kurosawa S, Mollica JS, Ferrell GL, Esmon CT. The endothelial cell protein C receptor augments protein C activation by the thrombin-thrombomodulin complex. Proc Natl Acad Sci USA. 1996;93:10212-6). 5x10⁴ cells/well were incubated in a 96-well plate with 0.02 U/ml of thrombin (0.17 nM)(ERL, Swansea, United Kingdom) and growing concentrations of PC (Baxter, Deerfield, IL, USA) between 50 and 1000 nM in Tris 20 mM buffer, pH 7.4, supplemented with NaCl 150 mM, CaCl₂ 5 mM, MgCl₂ 0.6 mM, 1% BSA, 0.001% Tween-20 and 0.02% NaN₃. After 45 minutes at room temperature, lepirudin was added (Schering AG, Berlin, Germany) at an end concentration of 0.2 µmol/L, to inhibit thrombin; 3-4 minutes later, chromogenic substrate S-2366 was added (Chromogenix, Milan, Italy) at an end concentration of 0.4 mM with the purpose of monitoring its proteolysis by APC. The increase in absorbance at 405 nm was recorded kinetically with a microplate reader (iEMS REader, Labsystems, Finland). Curve data fitting to the Michaelis-Menten equation was carried out using the Enzfitter program (Biosoft, Cambridge, United Kingdom), which calculated the Km of PC activation under those conditions. Where necessary, 45 µg/ml of purified anti-EPCR autoantibodies from patients (see below) were added simultaneously with the thrombin and PC. Thus, the effect of the anti-EPCR autoantibodies upon PC activation could be analyzed.

### Purification of anti-EPCR antibodies

### 1. Purification of IgM antibodies

One-ml samples of serum containing anti-EPCR autoantibodies were diluted in phosphate buffered saline (PBS) (sodium phosphate 100 mM, NaCl 0.15 M, pH=7.4) and filtered through a filter of 0.45 µm pore size. The filtrate was applied to a HitTrap column activated by NHS HP (Amersham Biosciences), where murine anti-human IgM polyclonal antibody had been previously immobilized (Maruyama S, Kubagawa H, Cooper MD. Activation of human B cells and inhibition of their terminal differentiation by monoclonal anti-murine antibodies. J Immunol. 1985;135:192-9). Human IgM was eluted with 5 ml of glycine 0.1 M, pH 2.5 and collected in 100 µl of Tris 1 M, pH 9.0. The fraction containing human IgM was concentrated and dialyzed against TB supplemented with CaCl₂ 5 mM and MgCl₂ 0.6 mM, pH 7.4.

### 2. Purification of IgA antibodies

One-ml samples of serum were diluted in PBS and manually applied to a jacaline column (Pierce). The adsorbed fraction was eluted with 2 ml of mellibiose 0.1 M in PBS and then dialyzed against PBS. Since posterior purification steps were required, the samples were applied to a HiTrap Protein G HP affinity column (Amersham Biosciences) to remove the contaminating IgG. The unbound product containing the IgA fraction was dialyzed against KH₂PO₄ 50 mM pH 7.0, and finally applied to a HiTrap Blue HP affinity column (Amersham Pharmacia Biotech) to remove albumin. The unbound material containing the purified IgA fraction was then collected and dialyzed against TB buffer supplemented with CaCl₂ 5 mM and MgCl₂ 0.6 mM, pH 7.4.

### 3. Purification of IgG antibodies

One-ml samples of serum containing anti-EPCR autoantibodies were diluted in phosphate buffered saline (PBS) (sodium phosphate 100 mM, NaCl 0.15 M, pH=7.4) and filtered through a filter of 0.45 µm pore size. The filtrate was applied to a HitTrap Protein G HP column (Amersham Biosciences). The human IgG was eluted with 5 ml of glycine 0.1 M, pH 2.5 and collected in 100 µl of Tris 1 M, pH 9.0. The fraction containing the human IgM was concentrated and dialyzed against TB supplemented with CaCl₂ 5 mM and MgCl₂ 0.6 mM, pH 7.4.

### Preparation of a rhsEPCR affinity column

rhsEPCR (2 mg in 3 ml of NaHCO₃ 100 mM, pH 8.5) was bound to a HitTrap NHS-activated HP affinity column (Amersham Biosciences) following the manufacturer's instructions. Once the reaction had been stopped with glycine 0.1 M, the rhsEPCR column was thoroughly washed with NaCl 2 M. This way the rhsEPCR column was able to bind PC in TBS, pH 7.4, supplemented with CaCl₂ 20 mM and MgCl₂ 0.6 mM. The PC could be eluted from the column with TBS supplemented with EDTA (data not shown). Since the rhsEPCR bound to the column maintained its capacity to bind PC, it could surely retain the native conformation and epitopes recognized by the autoantibodies. Consequently, the column thus prepared was adequate for eliminating anti-EPCR autoantibodies from a sample of serum or plasma.

### Statistical methods

In the study of APLS cases and controls, the comparison between patients (cases) and controls of the frequency of high levels of anti-EPCR IgM, IgA and IgG antibodies was carried out using the chi-squared test. The odds ratio (OR) (Martínez-González MA, of Irala-Estevez J & Guillén Grima F, (1999), 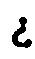 Qué es una odds ratio?, Medicina Clinica, 112, 11:416-422) and the 95% confidence interval (95%CI) were calculated as a measure of the association between APLS and anti-EPCR autoantibodies.

In the paired case-control study of fetal death, the comparison between cases and controls for continuous variables and by categories was carried out with the t-test for paired samples and with the McNemar test, respectively. The association between the levels of anti-EPCR autoantibodies corresponding to isotypes IgG and IgM with ACL and with anti-cardiolipin antibodies of IgM isotype was assessed based on the correlation coefficients for continuous variables and the Mann-Whitney test for variables by categories.

To evaluate the risk of fetal death associated with high levels of anti-EPCR autoantibodies of IgG and IgM isotypes, multiple regression analysis was used with case-control pairs. The principal independent variables were the levels of anti-EPCR autoantibodies corresponding to isotypes IgG and IgM by categories, according to the distribution of these immunoglobulins in controls. Different cutoff points were used to determine the levels associated to a higher risk. Uni- and multivariate analyses were carried out, fitting for known fetal death risk factors. It was not possible to include factors V Leiden (FVL) and ACL in the complete model; consequently, two models were considered for testing the effect of anti-EPCR autoantibodies:
(1) simultaneously introducing the levels of anti-EPCR autoantibodies corresponding to isotypes IgM and IgG, anti-cardiolipin antibody of IgM isotype, ACL and prothrombin G20210A; and
(2) identical to model 1, but adjusting for the presence/absence of FVL, instead of ACL.

Model (1) was used to evaluate the hypothesis that anti-cardiolipin antibodies and ACL are markers, rather than etiological factors, indicating a prothrombotic status caused by anti-EPCR autoantibodies. All calculations were made using SPSS, version 10.0 statistical package (SPSS Inc.).

### II. RESULTS

With the aim of investigating the presence of anti-EPCR autoantibodies in plasma and serum, rhsEPCR was first produced using the expression system of the yeast *P. pastoris.* Based on the described protocol, it was possible to purify more than 5 mg of rhsEPCR from a *P. pastoris* culture. Using electrophoresis in polyacrylamide with sodium dodecylsulfate (SDS-PAGE) gel and Western blot analysis with anti-myc monoclonal antibody, rhsEPCR appeared as a single and slightly heterogeneous band, reflecting the different degrees of glycosylation, as previously reported (Fukudome K, Kurosawa S, Stearns-Kurosawa DJ, He X, Rezaie AR, Esmon CT. The endothelial cell protein C receptor. Cell surface expression and direct ligand binding by the soluble receptor. J Biol Chem. 1996;271:17491-8)[see Figure 1].

The rhsEPCR was able to inhibit the anticoagulant activity of APC in the context of a coagulation test, as has been previously described (Regan LM, Stearns-Kurosawa DJ, Kurosawa S, Mollica J, Fukudome K, Esmon CT. The endothelial cell protein C receptor. Inhibition of activated protein C anticoagulant function without modulation of reaction with proteinase inhibitors. J Biol Chem. 1996;271:17499-503) (data not shown). In addition to binding PC with the expected affinity (see below), the activation of PC by thrombin on the surface of endothelial cells was characterized by a Km of 51±10 nM. The activation decreased considerably in the presence of rhsEPCR 2 µM (Km = 1000 nM approximately), implying a Ki of 70 nM approximately, and suggesting that rhsEPCR binds PC with similar efficacy to that of native EPCR, as has been previously reported (Fukudome K, Kurosawa S, Stearns-Kurosawa DJ, He X, Rezaie AR, Esmon CT. The endothelial cell protein C receptor. Cell surface expression and direct ligand binding by the soluble receptor. J Biol Chem. 1996;271:17491-8; Regan LM, Stearns-Kurosawa DJ, Kurosawa S, Mollica J, Fukudome K, Esmon CT. The endothelial cell protein C receptor. Inhibition of activated protein C anticoagulant function without modulation of reaction with proteinase inhibitors. J Biol Chem. 1996;271:17499-503). Such evidence is strongly suggestive of correct rhsEPCR activity and conformation - thus allowing its use for the detection of antibodies against human EPCR.

### Anti-EPCR autoantibodies in patients with APLS

Taking high levels to represent those above percentile 97 for each control group, high levels of anti-EPCR autoantibody corresponding to isotypes IgM, IgA or IgG were associated with APLS [OR = 4.47; 95%CI: 1.15-17.40](See Table 1). Extremely high levels of anti-EPCR autoantibodies (see Figure 2) were only detected in subjects diagnosed with APLS: three patients showed very high levels of anti-EPCR autoantibodies of IgM isotype (patient A = 407 AU, patient B = 301 AU, and patient C = 293 AU), two patients with APLS presented very high levels of anti-EPCR autoantibodies of IgA isotype (patient D = 795 AU and patient B = 475 AU, who also showed high levels of anti-EPCR autoantibodies of IgM isotype), and two patients presented high levels of anti-EPCR autoantibodies of IgG isotype (patient E = 230 AU and patient F = 220 AU). The 6 patients were women with a prior history of thrombosis - this being one of the selection criteria (stroke in patients A, C, D and F; cardiovascular disease in patient E; venous thrombosis in patients A, B, D and F). The most interesting observation is the fact that all the women presenting anti-EPCR autoantibodies of isotypes IgM and IgA (except patient B, who proved non-evaluable) suffered multiple episodes of fetal death.

In view of this finding, the analysis was directed towards the possible association between anti-EPCR autoantibodies and fetal death.

**Table 1**

| **OR of APLS associated with anti -EPCR antibodies** | | | |
|---|---|---|---|
| **Ariti-EPCR autoantibodies (percentile > 97%)** | **APLS (n = 43)** | **Control (n = 43)** | **OR (95%CI)** |
| IgG | | 1 | 3.10 (0.30-31.50) |
| IgM | 6 | 1 | 6.80 (0.80-59) |
| IgA | 3 | 1 | 3.10 (0.30-31.50) |
| IgG + IgM + IgA | 11 | 3 | 4.47 (1.15-17.4) |

### Biochemical characterization of anti-EPCR antibodies

The fractions of anti-EPCR autoantibodies of isotype IgM, Ig A and IgG from patients with extremely high levels were purified from 1 ml of serum. The fraction of anti-EPCR autoantibodies of IgM isotype in patient C was able to reduce the generation of APC by cultured endothelial cells in the presence of thrombin (20% of the residual capacity of PC activation, p = 0.02). The inhibitory effect was dose-dependent. In order to demonstrate that this effect of the fraction of anti-EPCR autoantibodies of IgM isotype in the patient with APLS was due to a specific antibody against EPCR, the sample was completely deprived of specific anti-EPCR autoantibody, loading it in an affinity column where rhsEPCR was immobilized. The fraction thus obtained lost its inhibitory action upon APC generation (87.6% of PC generation), which implies that the agent responsible for the phenomenon must have been a specific anti-EPCR autoantibody. None of the other fractions purified from patients with APLS were able to modify the capacity of endothelial cells to generate APC (Figure 3).

### Anti-EPCR autoantibodies in women with fetal death

The frequencies of the risk factors previously related to fetal death and of the anti-EPCR antibodies in the group of patients and controls are shown in Table 2.

**Table 2**

| **Univariate ORs and their confidence intervals for fetal death associated to the different variables studied** | | | | | |
|---|---|---|---|---|---|
| | **Fetal death (n=87)** | **Controls (n=87)** | **Paired OR** | **95%CI** | **P** |
| Anti-EPCR IgM | 16 | 3 | 14.0 | (1.8-106.4) | 0.01 |
| Anti-EPCR IgG | 13 | 4 | 4.3 | (12-15.2) | 0.02 |
| Factor V Leiden | 6 | 1 | 6.0 | (0.7-49.8) | 0.1 |
| Protein G20210A | 3 | 1 | 3.0 | (0.3-28.8) | 0.34 |
| ACL | 7 | 1 | 7.0 | (0.9-56.9) | 0.07 |
| Anticardiolipin IgM | 9 | 2 | 5.0 | (1.1-22.8) | 0.04 |
| Anticardiolipin IgG | 1 | 0 | - | - | - |

Percentile of 95% of the levels of anti-EPCR autoantibody of IgM isotype in the control group was 99 AU. Of the 87 patients, 16 (18%) presented values that exceeded this cutoff point, versus three subjects in the control group (n = 87). The OR not adjusted for fetal death in patients with levels of anti-EPCR autoantibodies of IgM isotype in excess of percentile 95 compared with those presenting a lower value was 14 (95% confidence interval (CI): 1.8-106.4). When the cutoff point was established at 90% percentile (83 AU), the OR was 5.2 (95%CI: 1.8-15.3).

Percentile 95 of the levels of anti-EPCR autoantibody of IgG isotype in the control group was 94 AU. Of the 87 patients, 13 (15%) showed values that exceeded this cutoff point, versus 4 subjects in the control group. The OR not adjusted for fetal death in patients with anti-EPCR autoantibodies of IgG isotype in excess of 95% percentile was 4.3 (95%CI: 1.2-15.2). When the cutoff point was established at a 90% (88.4 AU), the OR was 2.3 (95%CI: 0.9-5.6).

In addition, a multivariate analysis has been made adjusting for potential confounding factors. As commented above, it was not possible to include FVL and ACL in the same multivariate model - as a result of which two different models were considered: Model (1), adjusted for antiphospholipid antibodies (i.e., ACL and anticardiolipin antibodies) and prothrombin G20210A; and Model (2), including FVL but not ACL. The OR associated with anti-EPCR autoantibodies of IgM isotype in excess of percentile 95 in Model (1) was 23.1 (95%CI: 2-266.3) awhile in Model (2) the value was 31.0 (95%CI: 2-384.3). The OR associated with anti-EPCR autoantibodies of IgG isotype in excess of percentile 95 in Model (1) was 6.8 (95%CI: 1.2-38.4). According to Model (2), which includes factor V of Leiden instead of ACL, the OR associated with anti-EPCR autoantibodies of IgG isotype in excess of percentile 95 was 11.0 (95%CI: 1.6-73.5). The results are shown in Table 3.

**Table 3**

| **Multivariate ORs and their 95% confidence intervals for fetal death associated to high levels of anti-EPCR autoantibodies** | | | | | | |
|---|---|---|---|---|---|---|
| Anti-EPCR autoantibodies | **Model 1** | | | **Model 2** | | |
| | Paired OD | 95%CI | p* | Paired OD | 95%CI | p* |
| Anti-EPCR IgM | 23.0 | 2.0-266.3 | 0.012 | 31.0 | 2.0-384.3 | 0.007 |
| Anti-EPCR IgG | 6.8 | 1.2-38.4 | 0.029 | 11.0 | 1.6-73.5 | 0.013 |

These results indicate that anti-EPCR autoantibodies of isotypes IgM and IgG are independent risk factors for fetal death. However, high levels of IgA were not significantly associated to fetal death in this group of women.

### III. DISCUSSION

A method (specifically, an ELISA test) has been implemented that allows detection of the presence of autoantibodies against human EPCR. Using this system, a study has been made of a group of patients with APLS characterized by thrombosis and ACL, demonstrating (for the first time in human pathology) the presence of specific anti-EPCR autoantibodies of isotypes IgM, IgG and IgA. The study centered on the subgroup of patients with APLS and ACL because they have been associated with an increased risk of thrombosis; consequently, these subjects would be likely to present autoantibodies directly related to the clinical manifestations. In fact, many patients were found to have very high levels of anti-EPCR autoantibodies.

These autoantibodies could provide an explanation for the thrombosis and miscarriages seen in patients with APLS. Firstly, EPCR is a molecule expressed on the endothelium of large blood vessels and trophoblast. IgM and IgG immunoglobulins can bind and activate complement; if these antibodies are targeted to EPCR, they could activate complement on the endothelium and damage the latter - thus promoting thrombosis at this level. Secondly, it has been shown that the IgM fraction of a patient with high levels of anti-EPCR autoantibodies of IgM isotype can greatly reduce the generation of APC by endothelial cells in the presence of thrombin. This inhibitory effect disappears after specifically eliminating IgM targeted to EPCR by jointly passing the IgM fraction through an EPCR affinity column - which means that the inhibitory effect is due to an anti-EPCR autoantibody of IgM isotype. This antibody would probably result in low levels of APC *in vivo -* a situation in itself constituting a strong risk factor for thrombosis.

On selecting the patients according to the criterion of venous and/or arterial thrombosis, it did not prove possible to evaluate the risk of thrombosis associated with anti-EPCR autoantibodies. In contrast, increased levels of anti-EPCR autoantibodies, particularly of IgM isotype, were detected in women with a prior history of fetal death versus women without such antecedents. In view of these results in the pilot study, the decision was made to conduct a paired case-control study to evaluate the risk of a first episode of unexplainable fetal death in a general population of women associated with the presence of anti-EPCR autoantibodies. It was seen that high levels of anti-EPCR autoantibodies of IgM isotype (defined as a value in excess of percentile 95 of the value distribution in control subjects) constitute a strong risk factor for first episodes of fetal death, with a relative risk of 23 or 31, compared with lower levels. High levels of anti-EPCR autoantibodies of IgG isotype also constituted a strong risk factor, though less so than in the case of IgM isotype, with a relative risk of 7 or 11 - depending on the mathematical model used. In the univariate analysis, ACL and anticardiolipin antibody of IgM isotype were associated to an increased risk of fetal death, though this association was attenuated in the multivariate model - possibly because the information afforded by classical antiphospholipid antibodies is attributable to the associated anti-EPCR autoantibodies, which could represent an etiological factor of fetal death rather than a simple risk marker. Likewise, a study was made of the presence of FVL and prothrombin G20210A, which have recently been associated with an increased risk of late fetal death - an increased risk being identified in both the univariate and multivariate analyses in association to such polymorphism. The risk was not statistically significant, however, probably because of the number of patients included in the study.

In conclusion, this study for the first time demonstrates the presence of anti-EPCR autoantibodies in patients with APLS and thrombosis. The presence of anti-EPCR autoantibodies of IgM and IgG isotypes increases the risk of a first episode of fetal death. These autoantibodies may intrinsically contribute to thrombosis and to fetal death in patients with APLS and in the general population.

### EXAMPLE 2

### Detection of anti-EPCR autoantibodies in women with myocardial infarction

Study group: 142 women (aged 39±5 years, mean ± standard deviation) with myocardial infarction, and 142 healthy women (aged 39±5 years), matched by age and geographical origin. A study was made of the classical myocardial infarction risk factors (hypertension, hypercholesterolemia, diabetes, smoking and oral contraceptives). Assays were made of anti-EPCR autoantibodies IgG, IgM and IgA in samples of plasma, following the ELISA test protocol described under "Materials and Methods" (Example 1).
Results: High levels of anti-EPCR autoantibodies, defined by values in excess of percentile 93 of the distribution of the levels of anti-EPCR antibodies in the control group, were associated to an increased risk of myocardial infarction. In the multivariate analysis, high levels of anti-EPCR antibodies were associated to an adjusted odds ratio (OR) of 3.5, with a 95% confidence interval (95%CI) of 1.4-8.9 for IgA, while in the case of IgM the figures were OR = 3.0; 95%CI: 1.2-7.5.
Conclusion: High levels of anti-EPCR autoantibodies constitute an independent risk factor for myocardial infarction in women.

## Claims

1. A method to evaluate the presence of high levels of autoantibodies against endothelial PC / activated PC receptor (EPCR) in a sample, which comprises *in vitro* detecting and quantifying said autoantibodies against EPCR in said sample from a subject, by an immunological assay.

2. Method according to claim 1, wherein said sample is a sample of serum or plasma.

3. Method according to claim 1, wherein said subject is a human being.

4. Method according to claim 1, wherein the quantification of said anti-EPCR autoantibodies is carried out by means of an immunoassay coupled to a marker.

5. Method according to claim 1, wherein the quantification of said anti-EPCR autoantibodies is carried out by means of an ELISA test comprising:
a) immobilizing on a solid support a polypeptide comprising the EPCR amino acid sequence or a fragment thereof containing at least one epitope that can be recognized by an anti-EPCR autoantibody;
b) incubating the immobilized polypeptide with a sample suspected to contain anti-EPCR autoantibodies, obtained from the subject, for sufficient time to allow binding of the anti-EPCR autoantibodies to the immobilized polypeptide, and the formation of polypeptide-anti-EPCR autoantibody complexes;
c) removing the remaining sample not bound to the immobilized polypeptide; and
d) incubating the polypeptide-anti-EPCR autoantibody complexes with a second antibody conjugated to an enzyme, wherein said second antibody is able to bind to said anti-EPCR autoantibodies.

6. Method according to claim 5, wherein said polypeptide is selected from:
a) a polypeptide comprising the sequence of amino acids of full length EPCR; and
b) a polypeptide comprising the sequence of amino acids of a fragment of EPCR containing at least one epitope capable of being recognized by an anti-EPCR autoantibody.

7. Method according to any of claims 5 or 6, wherein said polypeptide is a fusion protein comprising:
a) a region A composed of a polypeptide containing the EPCR amino acid sequence or a fragment thereof containing at least one epitope capable of being recognized by an anti-EPCR autoantibody; and
b) a region B composed of a polypeptide comprising a sequence of amino acids of use for isolating or purifying the mentioned fusion protein, and/or a sequence of amino acids of use for anchoring the mentioned fusion protein to a solid support.

8. Method according to claim 7, wherein said region B is bound to the amino terminal end of region A.

9. Method according to claim 7, wherein said region B is bound to the carboxyl terminal end of region A.

10. Method according to any of claims 7 to 9, wherein said region A comprises the amino acid sequence of the soluble part of human EPCR.

11. Method according to any of claims 7 to 9, wherein the amino acid sequence of use for isolating or purifying the mentioned fusion protein, and/or an amino acid sequence of use for anchoring said fusion protein to a solid support present in region B, comprises a sequence of amino acids selected from Arg-tag, His-tag, FLAG-tag, Strep-tag, an epitope capable of being recognized by antibody, SBP-tag, S-tag, calmodulin binding peptide, cellulose binding domain, chitin binding domain, glutathione S-transferase-tag, maltose binding protein, NusA, TrxA, DsbA, Avi-tag, Ala-His-Gly-His-Arg-Pro (SEQ ID NO: 4) (2, 4, and 8 copies), Pro-Ile-His-Asp-His-Asp-His-Pro-His-Leu-Val-Ile-His-Ser (SEQ ID NO: 5), Gly-Met-Thr-Cys-X-X-Cys (SEQ ID NO: 6) (6 repetitions), β-galactosidase and VSV-glycoprotein.

12. Method according to any of claims 7 to 11, wherein said region B is composed of a polypeptide comprising a c-myc epitope capable of being recognized by an anti-c-myc antibody and a tail of histidines (His-tag).

13. Method according to any of claims 7 to 12, wherein said polypeptide is a fusion protein comprising the sequence of amino acids of the soluble part of human EPCR, the sequence of amino acids corresponding to c-myc epitope and a tail of histidines (His-tag).

14. Method according to any of claims 7 to 13, wherein said polypeptide is a fusion protein whose sequence of amino acids is shown in SEQ ID NO: 3.

15. Method according to claim 5, wherein said second antibody is an immunoglobulin isotype-specific antibody originating from a species different to that of the subject whose sample is being tested.

16. Method according to claim 5 or claim 15, wherein said second immunoglobulin isotype-specific antibody is selected from an anti-human IgG antibody, an anti-human IgM antibody, an anti-human IgA antibody, and their mixtures.

17. Method according to any of claims 15 or 16, wherein said second antibody is conjugated to an enzyme selected from between peroxidase and alkaline phosphatase.

18. Method according to any of claims 1 to 17, which further comprises the comparison of anti-EPCR autoantibody levels determined in the sample from the subject versus normal anti-EPCR autoantibody levels.

19. Method according to claim 1, wherein the variation in the levels of anti-EPCR autoantibodies is quantified over a given time period.

20. Method according to claim 19, wherein said sample originates from an individual previously diagnosed with an autoimmune or vascular disease, or who has suffered an obstetric complication, and is subject to therapeutic treatment.

21. Use of autoantibodies against endothelial PC/activated PC receptor (EPCR) as a marker in a method to evaluate the presence of high levels of autoantibodies against EPCR in a sample.

22. Use according to claim 21, wherein the presence of high levels of autoantibodies against EPCR in a sample is associated to a pathology selected from an autoimmune disease, a vascular disease and obstetric complications.

23. Use of a polypeptide comprising the endothelial PC/activated PC receptor (EPCR) amino acid sequence or a fragment thereof containing at least one epitope capable of being recognized by an anti-EPCR autoantibody in a method to determine the presence of high levels of autoantibodies against EPCR in a sample, said method comprising *in vitro* quantification of autoantibodies against EPCR in said sample.

24. Use according to claim 23, wherein said pathology related to the presence of high levels of autoantibodies against EPCR is selected from an autoimmune disease, a vascular disease and obstetric complications.

25. Use according to claim 23, wherein said polypeptide is a fusion protein comprising:
a) a region A composed of a polypeptide containing the EPCR amino acid sequence or a fragment thereof containing at least one epitope capable of being recognized by an anti-EPCR autoantibody; and
b) a region B composed of a polypeptide comprising a sequence of amino acids of use for isolating or purifying said fusion protein, and/or a sequence of amino acids of use for anchoring said fusion protein to a solid support.

26. Use of a polypeptide according to claim 25, wherein said region A comprises the amino acid sequence of the soluble part of human EPCR.

27. Use according to any of claims 25 or 26, wherein said polypeptide is a fusion protein comprising the amino acid sequence of the soluble part of human EPCR, the sequence of amino acids corresponding to c-myc epitope and a tail of histidines (His-tag).

28. Use of a polypeptide according to any of claims 25 to 27, wherein said polypeptide is a fusion protein with the amino acid sequence shown in SEQ ID NO: 3.

29. A kit for *in vitro* evaluation of the presence of high levels of autoantibodies against EPCR in a sample, said kit comprising a polypeptide that comprises the endothelial PC/activated PC receptor (EPCR) amino acid sequence or a fragment thereof containing at least one epitope capable of being recognized by an anti-EPCR autoantibody, and an antibody specific of a given immunoglobulin isotype, wherein said antibody is selected from an anti-human IgG antibody, an anti-human IgM antibody, and an anti-human IgA antibody and their mixtures.

30. Kit according to claim 29 3-4, wherein said polypeptide is a fusion protein comprising:
i) a region A composed of a polypeptide containing the EPCR amino acid sequence or a fragment thereof containing at least one epitope capable of being recognized by an anti-EPCR antibody; and
ii) a region B composed of a polypeptide comprising an amino acid sequence of use for isolating or purifying the mentioned fusion protein, and/or an amino acid sequence of use for anchoring the mentioned fusion protein to a solid support.

31. Kit according to claim 30, wherein said region A comprises the amino acid sequence of the soluble part of human EPCR.

32. Kit according to any of claims 30 or 31, wherein said polypeptide is a fusion protein comprising the amino acid sequence of the soluble part of human EPCR, the amino acid sequence corresponding to c-myc epitope and a tail of histidines (His-tag).

33. Kit according to any of claims 30 to 32, wherein said polypeptide is a fusion protein with the sequence of amino acids shown in SEQ ID NO: 3.

34. A method for detecting a pathology related to the presence of high levels of autoantibodies against the endothelial PC/activated PC receptor (EPCR) selected from an autoimmune disease, a vascular disease and obstetric complications, or for evaluating the risk and susceptibility of a subject to develop said pathology related to the presence of high levels of anti-EPCR autoantibodies in said subject, or for *in vitro* monitoring the effect of a therapy administered to a subject presenting said pathology related to the presence of high levels of anti-EPCR autoantibodies, which comprises:
- *in vitro* quantifying autoantibodies against the endothelial PC/activated PC receptor (EPCR) in a sample from said subject; and
- comparing the quantified levels of autoantibodies against EPCR in said sample to normal levels of anti-EPCR autoantibodies.

35. Method according to claim 34, wherein said autoimmune disease is selected from antiphospholipid syndrome, systemic lupus erythematosus, rheumatoid arthritis and autoimmune vasculitis.

36. Method according to claim 34, wherein said vascular disease is selected from arterial vascular disease, venous vascular disease and thrombosis of the microcirculation.

37. Method according to claim 36, wherein said vascular disease is selected from myocardial infarction, cerebral stroke, transient cerebrovascular accident, limb ischemia, atherosclerosis, aneurysm, thrombosis, superficial venous thrombosis, deep venous thrombosis, and pulmonary embolism.

38. Method according to claim 34, wherein said obstetric complication is selected from among miscarriage, fetal death, premature birth, delayed intrauterine growth, eclampsia and pre-eclampsia.

## Patentansprüche

1. Verfahren zur Bestimmung des Vorliegens von hohen Spiegeln von Autoantikörpern gegen endothelialen PC-/aktivierten PC-Rezeptor (EPCR) in einer Probe, das den *in vitro*-Nachweis und die Quantifizierung der Autoantikörper gegen EPCR in der Probe von einem Individuum mithilfe eines immunologischen Assays umfasst.

2. Verfahren nach Anspruch 1, wobei die Probe eine Serum- oder Plasmaprobe ist.

3. Verfahren nach Anspruch 1, wobei das Individuum ein Mensch ist.

4. Verfahren nach Anspruch 1, wobei die Quantifzierung der Anti-EPCR-Autoantikörper mithilfe eines mit einem Marker gekoppelten Immunassays durchgeführt wird.

5. Verfahren nach Anspruch 1, wobei die Quantifizierung der Anti-EPCR-Autoantikörper mithilfe eines ELISA-Tests durchgeführt wird, umfassend:
a) Immobilisieren eines Polypeptids, das die EPCR-Aminosäuresequenz umfasst, oder eines Fragments davon, das mindestens ein Epitop enthält, das von einem Anti-EPCR-Autoantikörper erkannt werden kann, auf einem festen Träger:
b) Inkubieren des immobilisierten Polypeptids mit einer von dem Individuum erhaltenen Probe, von der angenommen wird, dass sie Anti-EPCR-Autoantikörper enthält, über einen ausreichenden Zeitraum, um die Bindung der Anti-EPCR-Autoantikörper an das immobilisierte Polypeptid, und die Bildung von Polypeptid-Anti-EPCR-Autoantikörper-Komplexen zu ermöglichen;
c) Entfernen der restlichen nicht an das immobilisierte Polypeptid gebundenen Probe; und
d) Inkubieren der Polypeptid-Anti-EPCR-Autoantikörper-Komplexe mit einem zweiten an ein Enzym konjugierten Antikörper, wobei der zweite Antikörper fähig ist, an die Anti-EPCR-Autoantikörper zu binden.

6. Verfahren nach Anspruch 5, wobei das Polypeptid ausgewählt ist aus:
a) einem Polypeptid, das die Aminosäuresequenz des EPCRs voller Länge umfasst; und
b) einem Polypeptid, das die Aminosäuresequenz eines EPCR-Fragments umfasst, das mindestens ein Epitop enthält, das fähig ist, von einem Anti-EPCR-Autoantikörper erkannt zu werden.

7. Verfahren nach einem der Ansprüche 5 oder 6, wobei das Polypeptid ein Fusionsprotein ist, umfassend:
a) eine Region A, die aus einem Polypeptid gebildet ist, das die EPCR-Aminosäuresequenz enthält, oder einem Fragment davon, das mindestens ein zur Erkennung durch einen Anti-EPCR-Autoantikörper fähiges Epitop enthält; und
b) eine Region B, die aus einem Polypeptid gebildet ist, das eine Aminosäuresequenz umfasst, die zur Isolierung oder Aufreinigung des Fusionsproteins geeignet ist, und/oder eine Aminosäuresequenz, die zur Verankerung des Fusionsproteins auf einem festen Träger geeignet ist.

8. Verfahren nach Anspruch 7, wobei die Region B an den Amino-Terminus der Region A gebunden ist.

9. Verfahren nach Anspruch 7, wobei die Region B an den CarboxyTerminus der Region A gebunden ist.

10. Verfahren nach einem der Ansprüche 7 bis 9, wobei die Region A die Aminosäuresequenz des löslichen Teils von menschlichem EPCR umfasst.

11. Verfahren nach einem der Ansprüche 7 bis 9, wobei die in Region B vorliegende, zur Isolierung oder Aufreinigung des erwähnten Fusionsproteins geeignete Aminosäuresequenz und/oder die in Region B vorliegende, zur Verankerung des Fusionsproteins an einen festen Träger geeignete Aminosäuresequenz, eine Aminosäuresequenz umfasst/umfassen, die ausgewählt ist aus Arg-Tag, His-Tag, FLAG-Tag, Strep-Tag, einem Epitop, das fähig ist, von einem Antikörper erkannt zu werden, SBP-Tag, S-Tag, Calmodulin-bindendem Peptid, Cellulosebindender Domäne, Chitin-bindender Domäne, Glutathion-S-Transferase-Tag, Maltose-bindendem Protein, NusA, TrxA, DsbA, Avi-Tag, Ala-His-Gly-His-Arg-Pro (SEQ ID NO:4) (2, 4 und 8 Kopien), Pro-Ile-His-Asp-His-Asp-His-Pro-His-Leu-Val-Ile-His-Ser (SEQ ID NO:5), Gly-Met-Thr-Cys-X-X-Cys (SEQ ID NO:6) (6 Wiederholungen), β-Galactosidase und VSV-Glycoprotein.

12. Verfahren nach einem der Ansprüche 7 bis 11, wobei die Region B aus einem Polypeptid, das ein c-myc-Epitop umfasst, das fähig ist, von einem Anti-c-myc-Antikörper erkannt zu werden, und einem Histidin-Schwanz (His-Tag) gebildet ist.

13. Verfahren nach einem der Ansprüche 7 bis 12, wobei das Polypeptid ein Fusionsprotein ist, das die Aminosäuresequenz des löslichen Teils von menschlichem EPCR, die Aminosäuresequenz, die dem c-myc-Epitop entspricht und einem Histidin-Schwanz (His-Tag) umfasst.

14. Verfahren nach einem der Ansprüche 7 bis 13, wobei das Polypeptid ein Fusionsprotein ist, dessen Aminosäuresequenz in SEQ ID NO:3 dargestellt ist.

15. Verfahren nach Anspruch 5, wobei der zweite Antikörper ein Immunglobulinisotyp-spezifischer Antikörper ist, der von einer anderen Spezies als dem Individuum stammt, dessen Probe getestet wird.

16. Verfahren nach Anspruch 5 oder Anspruch 15, wobei der zweite Immunglobulinisotyp-spezifische Antikörper ausgewählt ist aus einem Anti-Human-IgG-Antikörper, einem Anti-Human-IgM-Antikörper, einem Anti-Human-IgA-Antikörper und Gemischen davon.

17. Verfahren nach einem der Ansprüche 15 oder 16, wobei der zweite Antiköper an ein Enzym konjugiert ist, das ausgewählt ist aus Peroxidase and alkalischer Phosphatase.

18. Verfahren nach einem der Ansprüche 1 bis 17, das des Weiteren den Vergleich der in der Probe von einem Individuum bestimmten Anti-EPCR-Autoantikörper-Spiegel mit normalen Anti-EPCR-Autoantikörper-Spiegeln umfasst.

19. Verfahren nach Anspruch 1, wobei die Abweichung der Anti-EPCR-Autoantikörper-Spiegel über einen bestimmten Zeitraum quantifiziert wird.

20. Verfahren nach Anspruch 19, wobei die Probe von einem Individuum stammt, bei dem zuvor eine Autoimmun- oder Gefäßerkrankung diagnostiziert wurde oder das eine Geburtskomplikation erlitten hat und eine therapeutische Behandlung erhält.

21. Verwendung von Autoantikörpern gegen endothelialen PC-/aktivierten PC-Rezeptor (EPCR) in einem Verfahren zur Bestimmung des Vorliegens hoher Spiegel von Autoantikörpern gegen EPCR in einer Probe.

22. Verfahren nach Anspruch 21, wobei das Vorliegen hoher Spiegel von Autoantikörpern gegen EPCR in einer Probe mit einem pathologischen Zustand in Zusammenhang steht, der ausgewählt ist aus einer Autoimmunerkrankung, einer Gefäßerkrankung und Geburtskomplikationen.

23. Verwendung eines Polypeptids, das die Aminosäuresequenz des endothelialen PC-/aktivierten PC-Rezeptors (EPCR) umfasst, oder eines Fragments davon, das mindestens ein zur Erkennung durch einen Anti-EPCR-Autoantikörper fähiges Epitop enthält, in einem Verfahren zur Bestimmung des Vorliegens hoher Spiegel von Anti-EPCR-Autoantikörpern, wobei das Verfahren die *in vitro*-Quantifizierung von Anti-EPCR-Autoantikörpem in der Probe umfasst.

24. Verwendung nach Anspruch 23, wobei der mit dem Vorliegen hoher Anti-EPCR-Autoantikörper-Spiegel in Zusammenhang stehende pathologische Zustand ausgewählt ist aus einer Autoimmunerkrankung, einer Gefäßerkrankung und Geburtskomplikationen.

25. Verwendung nach Anspruch 23, wobei das Polypeptid ein Fusionsprotein ist, umfassend:
a) eine Region A, die aus einem Polypeptid gebildet ist, das die EPCR-Aminosäuresequenz enthält, oder einem Fragment davon, das mindestens ein zur Erkennung durch einen Anti-EPCR-Autoantikörper fähiges Epitop enthält; und
b) eine Region B, die aus einem Polypeptid gebildet ist, das eine Aminosäuresequenz umfasst, die zur Isolierung oder Aufreinigung des Fusionsproteins geeignet ist, und/oder eine Aminosäuresequenz, die zur Verankerung des Fusionsproteins auf einem festen Träger geeignet ist.

26. Verwendung eines Polypeptids nach Anspruch 25, wobei die Region A die Aminosäuresequenz des löslichen Teils von menschlichem EPCR umfasst.

27. Verwendung nach einem der Ansprüche 25 oder 26, wobei das Polypeptid ein Fusionsprotein ist, das die Aminosäuresequenz des löslichen Teils von menschlichem EPCR, wobei die Aminosäuresequenz, die dem c-myc-Epitop entspricht und einem Histidin-Schwanz (His-Tag) umfasst.

28. Verwendung eines Polypeptids nach einem der Ansprüche 25 bis 27, wobei das Polypeptid ein Fusionsprotein mit der in SEQ ID NO:3 dargestellten Aminosäuresequenz ist.

29. Kit zur *in vitro*-Bestimmung des Vorliegens hoher Anti-EPCR-Autoantikörper in einer Probe, wobei der Kit ein Polypeptid umfasst, umfassend die Aminosäuresequenz des endothelialen PC-/aktivierten PC-Rezeptors (EPCR), oder ein Fragment davon, das mindestens ein zur Erkennung durch einen Anti-EPCR-Autoantikörper fähiges Epitop enthält, sowie einen für einen bestimmten Immunglobulinisotypen spezifischen Antikörper umfasst, wobei der Antikörper ausgewählt ist aus einem Anti-Human-IgG-Antikörper, einem Anti-Human-IgM-Antikörper und einem Anti-Human-IgA-Antikörper und Gemischen davon.

30. Kit nach Anspruch 29, wobei das Polypeptid ein Fusionsprotein ist, umfassend:
i) eine Region A, die aus einem Polypeptid gebildet ist, das die EPCR-Aminosäuresequenz enthält, oder ein Fragment davon, das mindestens ein zur Erkennung durch einen Anti-EPCR-antikörper fähiges Epitop enthält; und
ii) eine Region B, die aus einem Polypeptid gebildet ist, das eine Aminosäuresequenz umfasst, die zur Isolierung oder Aufreinigung des Fusionsproteins geeignet ist, und/oder eine Aminosäuresequenz, die zur Verankerung des Fusionsproteins auf einem festen Träger geeignet ist.

31. Kit nach Anspruch 30, wobei die Region A die Aminosäuresequenz des löslichen Teils von menschlichem EPCR umfasst.

32. Kit nach einem der Ansprüche 30 oder 31, wobei das Polypeptid ein Fusionsprotein ist, das die Aminosäuresequenz des löslichen Teils von menschlichem EPCR, die Aminosäuresequenz, die dem c-myc-Epitop entspricht und einem Histidin-Schwanz (His-Tag) umfasst.

33. Kit nach einem der Ansprüche 30 bis 32, wobei das Polypeptid ein Fusionsprotein mit der in SEQ ID NO:3 dargestellten Aminosäuresequenz ist.

34. Verfahren zum Nachweis eines pathologischen Zustands, der mit dem Vorliegen hoher Spiegel an Autoantikörpern gegen den endothelialen PC-/aktivierten PC-Rezeptor (EPCR) in Zusammenhang steht, ausgewählt aus einer Autoimmunerkrankung, einer Gefäßerkrankung und Geburtskomplikationen, oder zur Bestimmung des Risikos und Empfänglichkeit eines Individuums, den pathologischen Zustand zu entwickeln, der mit dem Vorliegen hoher Spiegel an Autoantikörpern gegen den endothelialen PC-/aktivierten PC-Rezeptor (EPCR) in diesem Individuum in Zusammenhang steht, oder zur *in vitro*-Überwachung der Wirkung einer Therapie, mit der ein Individuum behandelt wird, das einen pathologischen Zustand aufweist, der mit dem Vorliegen hoher Anti-EPCR-Autoantikörper in Zusammenhang steht, umfassend:
- in vitro-Quantifizierung von Autoantikörpern gegen den endothelialen PC-/aktivierten PC-Rezeptor (EPCR) in einer von dem Individuum stammenden Probe; und
- Vergleich der quantifizierten Anti-EPCR-Autoantikörper-Spiegel in der Probe mit normalen Anti-EPCR-Autoantikörper-Spiegeln.

35. Verfahren nach Anspruch 34, wobei die Autoimmunerkrankung ausgewählt ist aus Antiphospholipid-Syndrom, systemischem Lupus erythematodes, rheumatoider Arthritis und autoimmuner Vaskulitis.

36. Verfahren nach Anspruch 34, wobei die Gefäßerkrankung ausgewählt ist aus arteriellen Gefäßerkrankungen, venösen Gefäßerkrankungen und mikrovaskulären Thrombosen.

37. Verfahren nach Anspruch 36, wobei die Gefäßerkrankung ausgewählt ist aus Myokardinfarkt, Schlaganfall, transienter Apoplexie, GliedmaßenIschämie, Atherosklerose, Aneurysma, Thrombose, tiefer Venenthrombose, oberflächlicher Venenthrombose und Lungenembolie.

38. Verfahren nach Anspruch 34, wobei die Geburtskomplikation ausgewählt ist aus Fehlgeburt, fetalem Tod, Frühgeburt, intrauteriner Wachstumsverzögerung, Eklampsie und Präeklampsie.

## Revendications

1. Procédé pour évaluer la présence de teneurs élevées en autoanticorps dirigés contre le récepteur endothélial de la protéine C/la protéine C activée (EPCR) dans un échantillon, qui comprend la détection et la quantification *in vitro* des dits autoanticorps dirigés contre l'EPCR dans ledit échantillon provenant d'un sujet, par un essai immunologique.

2. Procédé selon la revendication 1, dans lequel ledit échantillon est un échantillon de sérum ou de plasma.

3. Procédé selon la revendication 1, dans lequel ledit sujet est un être humain.

4. Procédé selon la revendication 1, dans lequel la quantification des dits autoanticorps anti-EPCR est effectuée au moyen d'un essai immunologique combiné avec un marqueur.

5. Procédé selon la revendication 1, dans lequel la quantification des dits autoanticorps anti-EPCR est effectuée au moyen d'un test ELISA, comprenant:
a) l'immobilisation, sur un support solide, d'un polypeptide comprenant la séquence d'aminoacides d'EPCR ou un fragment de celle-ci, contenant au moins un épitope qui peut être reconnu par un autoanticorps anti-EPCR;
b) l'incubation du polypeptide immobilisé avec un échantillon suspecté de contenir des autoanticorps anti-EPCR, obtenu à partir du sujet, pendant un temps suffisant pour permettre la liaison des autoanticorps anti-EPCR au polypeptide immobilisé, et la formation de complexes polypeptide-autoanticorps anti-EPCR ;
c) l'élimination de l'échantillon restant non lié au polypeptide immobilisé ; et
d) l'incubation des complexes polypeptide-autoanticorps anti-EPCR avec un deuxième anticorps conjugué avec une enzyme, dans lequel ledit deuxième anticorps est apte à se lier aux dits autoanticorps anti-EPCR.

6. Procédé selon la revendication 5, dans lequel ledit polypeptide est choisi parmi :
a) un polypeptide comprenant la séquence d'aminoacides de l'EPCR de longueur entière ; et
b) un polypeptide comprenant la séquence d'aminoacides d'un fragment de EPCR contenant au moins un épitope pouvant être reconnu par un autoanticorps anti-EPCR.

7. Procédé selon l'une quelconque des revendications 5 ou 6, dans lequel ledit polypeptide est une protéine fusionnée, comprenant :
a) une région A composée d'un polypeptide contenant la séquence d'aminoacides de l'EPCR ou un fragment de celle-ci, contenant au moins un épitope pouvant être reconnu par un autoanticorps anti-EPCR ; et
b) une région B composée d'un polypeptide comprenant une séquence d'aminoacides, destinée à être utilisée pour isoler ou purifier la protéine fusionnée mentionnée, et/ou une séquence d'aminoacides, destinée à être utilisée pour ancrer la protéine fusionnée mentionnée à un support solide.

8. Procédé selon la revendication 7, dans lequel ladite région B est liée à l'extrémité amino-terminale de la région A.

9. Procédé selon la revendication 7, dans lequel ladite région B est liée à l'extrémité carboxy-terminale de la région A.

10. Procédé selon l'une quelconque des revendications 7 à 9, dans lequel ladite région A comprend la séquence d'aminoacides de la partie soluble de l'EPCR humain.

11. Procédé selon l'une quelconque des revendications 7 à 9, dans lequel la séquence d'aminoacides destinée à être employée pour isoler ou purifier la protéine fusionnée mentionnée, et/ou une séquence d'aminoacides destinée à être employée pour ancrer ladite protéine fusionnée à un support solide, présente dans la région B, comprennent une séquence d'aminoacides choisie parmi Arg-tag, His-tag, FLAG-tag, Strep-tag, un épitope pouvant être reconnu par un anticorps, SBP-tag, S-tag, un peptide de liaison à la calmoduline, un domaine de liaison à la cellulose, un domaine de liaison à la chitine, le glutathion S-transférase-tag, une protéine de liaison au maltose, NusA, TrxA, DsbA, Avi-tag, Ala-His-Gly-His-Arg-Pro (SEQ ID N° : 4) (2, 4 et 8 copies), Pro-Ile-His-Asp-His-Asp-His-Pro-His-Leu-Val-Ile-His-Ser (SEQ ID N° : 5), Gly-Met-Thr-Cys-X-X-Cys (SEQ ID N° : 6) (6 répétitions), la β-galactosidase et la glycoprotéine de VSV.

12. Procédé selon l'une quelconque des revendications 7 à 11, dans lequel ladite région B est composée d'un polypeptide comprenant un épitope c-myc pouvant être reconnu par un anticorps anti-c-myc, et une queue d'histidines (His-tag).

13. Procédé selon le quelconque des revendications 7 à 12, dans lequel ledit polypeptide est une protéine fusionnée comprenant la séquence d'aminoacides de la partie soluble de l'EPCR humain, la séquence d'aminoacides correspondant à l'épitope c-myc, et une queue d'histidines (His-tag).

14. Procédé selon l'une quelconque des revendications 7 à 13, dans lequel ledit polypeptide est une protéine fusionnée dont la séquence d'aminoacides est illustrée par la SEQ ID N° : 3.

15. Procédé selon la revendication 5, dans lequel ledit deuxième anticorps est un anticorps spécifique d'isotype d'immunoglobuline, provenant d'une espèce différente de celle du sujet dont un échantillon est testé.

16. Procédé selon la revendication 5 ou la revendication 15, dans lequel ledit deuxième anticorps spécifique d'isotype d'immunoglobuline est choisi parmi un anticorps IgG anti-humain, un anticorps IgM anti-humain, un anticorps IgA anti-humain et leurs mélanges.

17. Procédé selon l'une quelconque des revendications 15 ou 16, dans lequel ledit deuxième anticorps est conjugué avec une enzyme choisie parmi la peroxydase et la phosphatase alcaline.

18. Procédé selon l'une quelconque des revendications 1 à 17, qui comprend, en outre, la comparaison des teneurs d'autoanticorps anti-EPCR, déterminés dans l'échantillon provenant du sujet, avec des teneurs d'autoanticorps anti-EPCR normaux.

19. Procédé selon la revendication 1, dans lequel la variation des teneurs d'autoanticorps anti-EPCR est quantifiée pendant une période de temps donnée.

20. Procédé selon la revendication 19, dans lequel ledit échantillon provient d'un individu auquel on a préalablement diagnostiqué une maladie auto-immune ou vasculaire, ou qui a souffert d'une complication obstétricale, et est soumis à un traitement thérapeutique.

21. Utilisation d'autoanticorps dirigés contre le récepteur endothélial de la protéine C/la protéine C activée (EPCR) dans un procédé pour évaluer la présence de teneurs élevées en autoanticorps dirigés contre l'EPCR dans un échantillon.

22. Utilisation selon la revendication 21, dans laquelle la présence de teneurs élevées en autoanticorps dirigés contre l'EPCR dans un échantillon, est associée à une pathologie choisie parmi une maladie auto-immune, une maladie vasculaire et les complications obstétricales.

23. Utilisation d'un polypeptide comprenant la séquence d'aminoacides du récepteur endothélial de la protéine C/protéine C activée (EPCR) ou un fragment de celle-ci, contenant au moins un épitope pouvant être reconnu par un autoanticorps anti-EPCR, dans un procédé pour déterminer la présence de teneurs élevées en autoanticorps dirigés contre l'EPCR dans un échantillon, ledit procédé comprenant la quantification *in vitro* des autoanticorps dirigés contre l'EPCR dans ledit échantillon.

24. Utilisation selon la revendication 23, dans laquelle ladite pathologie liée à la présence de teneurs élevées en autoanticorps dirigés contre l'EPCR, est choisie parmi une maladie auto-immune, une maladie vasculaire et les complications obstétricales.

25. Utilisation selon la revendication 23, dans laquelle ledit polypeptide est une protéine fusionnée, comprenant:
a) une région A composée d'un polypeptide contenant la séquence d'aminoacides de l'EPCR ou un fragment de celle-ci, contenant au moins un épitope pouvant être reconnu par un autoanticorps anti-EPCR ; et
b) une région B composée d'un polypeptide comprenant une séquence d'aminoacides, destinée à être utilisée pour isoler ou purifier la protéine fusionnée mentionnée, et/ou une séquence d'aminoacides, destinée à être utilisée pour ancrer la protéine fusionnée mentionnée à un support solide.

26. Utilisation d'un polypeptide selon la revendication 25, dans laquelle ladite région A comprend la séquence d'aminoacides de la partie soluble de l'EPCR humain.

27. Utilisation selon l'une quelconque des revendications 25 ou 26, dans laquelle ledit polypeptide est une protéine fusionnée comprenant la séquence d'aminoacides de la partie soluble de l'EPCR humain, la séquence d'aminoacides correspondant à l'épitope c-myc, et une queue d'histidines (His-tag).

28. Utilisation d'un polypeptide selon l'une quelconque des revendications 25 à 27, dans laquelle ledit polypeptide est une protéine fusionnée dont la séquence d'aminoacides est illustrée par la SEQ ID N° : 3.

29. Kit pour l'évaluation *in vitro* de la présence de teneurs élevées en autoanticorps dirigés contre l'EPCR dans un échantillon, ledit kit comprenant un polypeptide qui comprend la séquence d'aminoacides du récepteur endothélial de la protéine C/la protéine C activée (EPCR) ou un fragment de celle-ci contenant au moins un épitope pouvant être reconnu par un autoanticorps anti-EPCR, et un anticorps spécifique d'un isotype d'immunoglobuline donné, dans lequel ledit anticorps est choisi parmi un anticorps IgG anti-humain, un anticorps IgM anti-humain, un anticorps IgA anti-humain et leurs mélanges.

30. Kit selon la revendication 29, dans lequel ledit polypeptide est une protéine fusionnée, comprenant :
i) une région A composée d'un polypeptide contenant la séquence d'aminoacides de l'EPCR ou un fragment de celle-ci, contenant au moins un épitope pouvant être reconnu par un autoanticorps anti-EPCR ; et
ii) une région B composée d'un polypeptide comprenant une séquence d'aminoacides, destinée à être utilisée pour isoler ou purifier la protéine fusionnée mentionnée, et/ou une séquence d'aminoacides, destinée à être utilisée pour ancrer la protéine fusionnée mentionnée à un support solide.

31. Kit selon la revendication 30, dans lequel ladite région A comprend la séquence d'aminoacides de la partie soluble de l'EPCR humain.

32. Kit selon le quelconque des revendications 30 ou 31, dans lequel ledit polypeptide est une protéine fusionnée comprenant la séquence d'aminoacides de la partie soluble de l'EPCR humain, la séquence d'aminoacides correspondant à l'épitope c-myc, et une queue d'histidines (His-tag).

33. Kit selon l'une quelconque des revendications 30 à 32, dans lequel ledit polypeptide est une protéine fusionnée dont la séquence d'aminoacides est illustrée par la SEQ ID N° : 3.

34. Procédé pour la détection d'une pathologie liée à la présence de teneurs élevées en autoanticorps dirigés contre le récepteur endothélial de la protéine C/la protéine C activée (EPCR), choisie parmi une maladie auto-immune, une maladie vasculaire et les complications obstétricales, ou pour évaluer le risque et la propension d'un sujet à développer ladite pathologie liée à la présence de teneurs élevées en autoanticorps anti-EPCR chez ledit sujet, ou pour contrôler, *in vitro,* l'effet d'une thérapie administrée à un sujet présentant ladite pathologie liée à la présence de teneurs élevées en auto anticorps anti-EPCR, qui comprend :
- la quantification, *in vitro*, des autoanticorps dirigés contre le récepteur endothélial de la protéine C/la protéine C activée (EPCR) dans un échantillon provenant du ledit sujet ; et
- la comparaison des teneurs quantifiées d'autoanticorps dirigés contre l'EPCR dans ledit échantillon, à des teneurs normales en autoanticorps anti-EPCR.

35. Procédé selon la revendication 34, dans lequel ladite maladie auto-immune est choisie parmi le syndrome des antiphospholipides, le lupus érythémateux disséminé, l'arthrite rhumatoïde et la vasculite auto-immune.

36. Procédé selon la revendication 34, dans lequel ladite maladie vasculaire est choisie parmi une maladie vasculaire artérielle, une maladie vasculaire veineuse et la thrombose de la microcirculation.

37. Procédé selon la revendication 36, dans lequel ladite maladie vasculaire est choisie parmi l'infarctus du myocarde, un ictus cérébral, un accident cérébrovasculaire transitoire, l'ischémie d'un membre, l'athérosclérose, un anévrisme, la thrombose, la thrombose veineuse superficielle, la thrombose veineuse profonde et l'embolie pulmonaire.

38. Procédé selon la revendication 34, dans lequel ladite complication obstétricale est choisie parmi une fausse couche, une mort foetale, une naissance prématurée, une croissance intra-utérine retardée, l'éclampsie et la pré-éclampsie.


### LIST OF SEQUENCES

<110> Proyecto de Biomedicina CIMA, S.L.
<120> METHOD FOR EVALUATING RISK AND SUSCEPTIBILITY OF DEVELOPING A PATHOLOGY RELATED TO THE PRESENCE OF AUTOANTIBODIES AGAINST EPCR
<160> 3
<170> PatentIn version 2.0
<210> 1
   <211> 36
   <212> DNA
   <213> Artificial sequence
   <223> Primer oligonucleotide
<400> 1
   agcttggcat atcgattagc caagacgcct cagatg 36
<210> 2
   <211> 36
   <212> DNA
   <213> Artificial sequence
   <223> Primer oligonucleotide
<400> 2
   tattctatgc ggccgccgaa gtgtaggagc ggcttg 36
<210> 3
   <211> 36
   <212> PRT
<400> 3
<210> 4
   <211> 6
   <212> PRT
   <213> artificial sequence
<220>
   <223> anchoring protein 1
<400> 4
   Ala His Gly His Arg Pro
   1 5
<210> 5
   <211> 14
   <212> PRT
   <213> Artificial sequence
<220>
   <223> Anchoring protein
<400> 5
   Pro Ile His Asp His Asp His Pro His Leu Val Ile His Ser
   1 5 10
<210> 6
   <211> 7
   <212> PRT
   <213> Artificial sequence
<220>
   <223> Anchoring protein 3
<220>
   <221> MISC. FEATURE
   <222> (5)..(6)
   <223> essential amino acid
<400> 6
   Gly Met Thr Cys Xaa Xaa Cys
   1               5